# DEMANDE DE BREVET EUROPEEN

(11) **EP 2 923 735 A1**
(43) Date de publication de la demande: **30.09.2015**
(21) Numéro de dépôt: 15160808.0
(22) Date de dépôt: 25.03.2015
(51) Int. Cl.: A61Q 3/02, A61K 8/73, A61K 8/85

(54) **Composition cosmétique anhydre applicable sur les ongles**

(30) Priorité: 26.03.2014 LU 92414
(71) Demandeur: International Lacquers S.A., 3225 Bettembourg (LU)
(72) Inventeur: Dylewicz, Carole, 57100 Thionville Garche (FR); Armbruster, Marc, 54920 Villers-la-Montagne (FR)
(74) Mandataire: Office Freylinger

(57) **Abrégé**

La présente invention concerne une composition anhydre pour vernis à ongles comprenant au moins un solvant organique cosmétiquement acceptable, au moins un agent filmogène et une résine de copolymère(s) d'acide adipique, d'acide fumarique, de néopentylglycol et de cyclohexanediméthanol, de préférence une résine de Polyester-26, et son procédé de préparation. Un autre aspect de l'invention concerne l'utilisation de ces résines pour améliorer les propriétés d'étalement et d'application d'un vernis à ongles.

## Description

### Domaine technique

La présente invention concerne une composition cosmétique anhydre applicable sur les ongles ou les faux ongles formant un film dur, brillant et tenace, ainsi que son procédé de préparation.

### Etat de la technique

Les vernis à ongles sont généralement utilisés pour maquiller les ongles ainsi que les faux ongles. Les vernis à ongles sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants.

Pour répondre aux exigences des utilisateurs, les vernis à ongles doivent satisfaire plusieurs critères :
- Une bonne application, lorsque le vernis à ongles est appliqué sur l'ongle, il doit former une surface lisse, sans stries avec une épaisseur constante afin d'obtenir un film et une coloration homogènes,
- Un étalement facilité sur toute la surface de l'ongle pour rendre son utilisation pratique,
- Une brillance accrue et qui persiste dans le temps,
- Un temps de séchage et de durcissement sur l'ongle très courts,
- Une bonne tenue du vernis à ongles sur l'ongle, il est en effet souhaité que le vernis s'écaille le moins possible afin de conserver un aspect esthétique.

De nouvelles formulations sont sans cesse développées pour essayer d'améliorer l'une ou l'autre de ces propriétés.

Par exemple, concernant la tenue des vernis à ongles, ils présentent pour la plupart une tenue très limitée dans le temps et s'écaillent en général au bout de quelques jours. Il en résulte que les utilisateurs doivent répéter fréquemment l'application de vernis à ongles ce qui peut être perçue comme fastidieuse.

Récemment, des vernis à ongles présentant une longue tenue dans le temps dits de longue tenue, encore appelés semi permanents ou « gel like » ont été développés.

Ces vernis à ongles peuvent présenter une tenue sur l'ongle jusqu'à 15 jours. Néanmoins, ces vernis à ongles ont l'inconvénient d'être difficiles à étaler sur l'ongle. De ce fait l'application de ces vernis peut s'avérer médiocre et aboutir à la formation d'un film non-homogène sur l'ongle. Ainsi, bien que sa tenue soit améliorée, son application est quant à elle plus mauvaise. Il apparaît donc difficile d'améliorer une propriété du vernis à ongles sans en affecter une autre. Dans ce cas particulier, l'amélioration de la tenue du vernis à ongles peut impacter de façon négative la pose du vernis à ongles en la rendant contraignante.

De façon plus générale, quel que soit le type de vernis à ongles, l'étalement et l'application d'un vernis sont deux critères importants, car il conditionne la pose du vernis à ongles. En effet, plus l'étalement et l'application d'un vernis à ongles sont médiocres, plus sa pose sera chronophage et fastidieuse.

Les vernis à ongles présentant une pose rapide et pratique sont actuellement très plébiscités par les utilisateurs. Cependant, ces vernis à ongles doivent également satisfaire à tous les autres critères requis par les utilisateurs : brillance, couvrance, tenue, temps de séchage et de durcissement courts. L'amélioration de l'application du vernis ne doit pas être obtenue au détriment des autres propriétés.

En conséquence, il existe un besoin de développer des vernis à ongles dont l'étalement et l'application sont améliorés de sorte que la pose des vernis soit la plus commode possible. En particulier, il existe une réelle demande de fournir des agents d'amélioration de l'application et de l'étalement du vernis à ongles compatibles avec tous les types de formulations de vernis à ongles, sans altérer (significativement) leurs autres propriétés.

### Objet de l'invention

Un objet de la présente invention est de fournir une composition anhydre cosmétique pour vernis à ongles présentant d'excellentes propriétés d'application et d'étalement sur l'ongle tout en maintenant ses autres propriétés, à savoir : la brillance, la tenue, la couvrance ainsi qu'un temps de séchage et de durcissement courts.

Conformément à l'invention, cet objectif est atteint par une composition anhydre pour vernis à ongles selon la revendication 1.

### Description générale de l'invention

Afin de résoudre le problème mentionné ci-dessus, la présente invention fournit une composition anhydre pour vernis à ongles comprenant au moins un solvant organique cosmétiquement acceptable, au moins un agent filmogène et une résine à base d'un ou de plusieurs copolymères d'acide adipique, d'acide fumarique, de néopentylglycol et de cyclohexanediméthanol. De préférence, le ou les copolymères sont des copolymères statistiques. Parmi ces copolymères, le Polyester-26 (Polyester-26, désignation INCI) est particulièrement préféré.

Dans une variante particulièrement préférée, la composition anhydre pour vernis à ongles selon l'invention comprend une résine à base d'un ou de plusieurs copolymères d'acide adipique, d'acide fumarique, de néopentylglycol et de cyclohexanediméthanol solubilisée dans un solvant et en particulier une résine de Polyester-26 solubilisée dans un solvant. De préférence, le solvant utile pour solubiliser la résine à base d'un ou de plusieurs copolymères d'acide adipique, d'acide fumarique, de néopentylglycol et de cyclohexanediméthanol et en particulier la résine de Polyester-26, est l'acétate de butyle. Préférablement, la résine est solubilisée dans le solvant dans les proportions suivantes :
- 60 % en poids d'une résine à base d'un ou de plusieurs copolymères d'acide adipique, d'acide fumarique, de néopentylglycol et de cyclohexanediméthanol, de préférence d'une résine de Polyester-26 et
- 40 % en poids de solvant, de préférence d'acétate de butyle, par rapport au poids total de la résine solubilisée dans le solvant.

De façon surprenante, il a été mis en évidence que la résine de copolymères d'acide adipique, d'acide fumarique, de néopentylglycol et de cyclohexanediméthanol, en particulier la résine de Polyester-26, permettait d'améliorer considérablement les propriétés d'étalement et d'application du vernis à ongles. L'utilisation de ces résines dans une composition anhydre pour vernis à ongles conduit alors à une pose rapide et pratique du vernis à ongles. De surcroît, l'ajout de ces résines n'affecte pas les autres propriétés de la composition anhydre pour vernis à ongles. Ainsi, l'utilisation de ces résines de copolymères d'acide adipique, d'acide fumarique, de néopentylglycol et de cyclohexanediméthanol, en particulier la résine de Polyester-26, conduit à l'obtention d'une composition anhydre pour vernis à ongle facile à appliquer sur l'ongle et qui de surcroît présente une tenue, une brillance, une couvrance ainsi qu'un temps de séchage et de durcissement avantageux.

En outre, l'utilisation de résine de copolymères d'acide adipique, d'acide fumarique, de néopentylglycol et de cyclohexanediméthanol, en particulier la résine de Polyester-26, présente l'avantage qu'il offre une très bonne compatibilité avec les autres composants habituellement utilisés dans les compositions anhydres. Il peut donc être utilisé dans tous les types de compositions anhydres pour vernis à ongles comme par exemple les vernis à ongles à effet (miroir, craquelé, irisé, martelé, magnétique, mat, métallisé etc...), les vernis à ongles longue tenue (« gel like »), mais également les vernis à ongles dits « top coat » ou « base coat » (destinés à être appliqués sur ou en dessous d'un vernis à ongles). Dans tous les cas, l'utilisation de résine de copolymères d'acide adipique, d'acide fumarique, de néopentylglycol et de cyclohexanediméthanol, en particulier de la résine de Polyester-26, résultera en une amélioration significative des propriétés d'étalement et d'application du vernis sur l'ongle sans pour autant affecter les autres propriétés (esthétiques ou autres) du vernis à ongles. Par exemple, dans le cas d'une formulation « gel like », la tenue du vernis à ongles ne sera pas affectée par l'ajout de la résine de copolymères d'acide adipique, d'acide fumarique, de néopentylglycol et de cyclohexanediméthanol, en particulier de la résine de Polyester-26.

De façon préférée, la composition anhydre pour vernis à ongle comprend une quantité de résine de copolymères d'acide adipique, d'acide fumarique, de néopentylglycol et de cyclohexanediméthanol, en particulier la résine de Polyester-26, comprise entre 0,1% et 5% en poids sec par rapport au poids total de la composition et de façon encore plus préférée entre 0,3 % et 3 % en poids sec par rapport au poids total de la composition.

Selon un mode de réalisation de l'invention, la composition anhydre pour vernis à ongles comprend un solvant organique, en plus du solvant éventuellement utilisé pour solubiliser la résine de copolymères d'acide adipique, d'acide fumarique, de néopentylglycol et de cyclohexanediméthanol, en particulier la résine de Polyester-26. Ce solvant organique peut être choisi parmi le groupe constitué par le toluène, le xylène, l'acétate d'éthyle, l'acétate de butyle, l'acétate d'isobutyle, l'acétate de propyle, l'acétate de méthyle, une cétone, un ester, l'isopropanol, le butanol, le diacétone alcool, l'éthanol dénaturé (avec la méthylethylcétone ou le diethylether), les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition anhydre pour vernis à ongles. En outre de façon préférée, la quantité de solvant organique présente dans la composition anhydre pour vernis à ongles est comprise entre 60% et 80% en poids par rapport au poids total de la composition.

Avantageusement, le solvant organique est l'acétate de butyle et/ou l'acétate d'éthyle.

Le ou les agent(s) filmogène(s) compris dans la composition anhydre pour vernis à ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxypropyl-cellulose. Préférentiellement, on choisira la nitrocellulose comme agent filmogène.

Un agent filmogène seul ou une combinaison d'agents filmogènes peut être utilisé(e) dans la composition anhydre pour vernis à ongles dans les proportions allant de 10 % à 20 % en poids sec et de façon préférée de 12 % à 16 % en poids sec par rapport au poids total de la composition.

En plus, de la résine de copolymères d'acide adipique, d'acide fumarique, de néopentylglycol et de cyclohexanediméthanol, en particulier la résine de Polyester-26, des résines supplémentaires (différentes) peuvent également être ajoutées dans la composition. En particulier, ces résines supplémentaires permettent d'augmenter le collant et l'adhérence du film sur l'ongle.

Les résines pouvant être utilisées sont les suivantes :
- Les résines tosylamide/formaldéhyde ou résines toluènesulfonamide/formaldéhyde,
- Les résines tosylamide/époxy ou résines toluènesulfonamide/époxy,
- Copolymères acide adipique/néopentyl glycol/anhydride trimellitique,
- Copolymères d'anhydride phtalique/glycérine/glycidyl décanoate,
- Copolymères d'anhydride phtalique/anhydride trimellitique/glycols,
- Copolymères de glycérine/acide phtalique,
- Copolymères styrène/acrylate/acrylonitrile,
- Polymères et copolymères d'acrylates,
- Copolymères d'acrylates et de styrène,
- Sucrose acétate isobutyrate,
- Résine polyvinylbutyral.

En plus de leur action collante, la plupart de ces résines additionnelles ont également une action plastifiante. Les résines additionnelles peuvent être utilisées dans la formule seules ou sous la forme de mélanges dans des quantités allant de 3 à 20 % en poids sec, de préférence 5 à 12% en poids sec, en particulier de 7 à 10 % en poids sec par rapport au poids total de la composition.

Selon un mode de réalisation, la composition anhydre pour vernis à ongles peut également comprendre des plastifiants. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 2 à 12 % en poids sec, de préférence 5 à 10 % en poids sec, en particulier de 6 à 8 % en poids sec par rapport au poids total de la composition.

Des exemples de plastifiants utilisables dans l'invention sont l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phthalate, le diisobutyl adipate, le diisopropyl adipate, le dipropylène glycol dibenzoate, etc...

Pour colorer ou fournir des effets particuliers à la composition anhydre pour vernis à ongles, elle peut comprendre un ou plusieurs agents de coloration choisis parmi les pigments, les nacres, les glitters (paillettes) et/ou les particules métalliques. Ces agents de coloration fournissent l'aspect esthétique recherché par les utilisateurs de vernis à ongles. Ces agents de coloration peuvent produire des effets très différents tels que « irisé », « métallisé », « nacré », « miroir », « crème », « pailleté », craquelé », « mat », « néon », « fluorescent », « holographique », etc.

En particulier, les pigments utilisés peuvent être sélectionnés parmi le groupe constitué par le dioxyde de titane (CI 77891), l'oxyde de fer noir (CI 77499), l'oxyde de fer rouge (CI 77491), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Les nacres sont également utilisables dans l'invention, les nacres sont des particules minérales de différentes natures recouvertes d'une ou plusieurs couches d'oxydes (oxydes de titane ou oxydes de fer) ou encore de pigments. Les nacres peuvent se présenter sous la forme d'une poudre ou de plaque de tailles variées. Parmi les nacres utilisables dans l'invention, on peut citer :
- les nacres préparées à partir de mica naturel et ayant subi des opérations de coating successives,
- les nacres préparées à partir de mica synthétique appelé également « synthetic fluorphlogopite »,
- les nacres préparées à partir de calcium sodium borosilicate,
- les nacres préparées à partir d'oxyde de silice.

Les glitters (paillettes) utilisables dans l'invention peuvent être préparés à partir de plusieurs types de matériaux comme les films polyesters comportant en surface une couche métallique, le polyéthylène téréphtalate, le polybutylène téréphtalate, les copolymères d'acrylates, les copolymères d'acrylates ou encore l'aluminium.

Selon un mode de réalisation de l'invention, la composition anhydre pour vernis à ongles comprend une quantité d'agent de coloration comprise de 0,1 % à 20 % en poids sec et de préférence de 0,5 % à 12 % en poids sec par rapport au poids total de la composition.

La composition anhydre pour vernis à ongles peut en outre comprendre divers additifs choisis parmi les agents filmogènes, les résines, les agents rhéologiques, les absorbeurs UV, les modificateurs de surface et les agents dits « traitants ».

Les différents additifs utilisables dans l'invention sont :

Parmi les additifs de rhéologie, on citera notamment les dérivés d'argile modifiés comme le stéaralkonium hectorite ou le stéaralkonium bentonite. Ces additifs de rhéologie seront de préférence utilisés de 0,1 à 3 % en poids sec par rapport au poids total de la composition. Ces additifs permettent notamment la suspension des particules insolubles dans le vernis tels que les pigments ou encore les nacres.

Parmi les absorbants UV, on citera les absorbants de lumière UV qui permettent de protéger la formulation ou les agents colorants utilisés l'invention des rayonnements UV. Parmi les protecteurs UV utilisables dans l'invention, on citera : la benzophénone-1 (CAS 131-56-6), la benzophénone-3 (CAS 131-57-7), le benzyl salicylate (CAS 118-58-1), l'étocrylène (CAS 5232-99-5), le drométrizole (CAS 2440-22-4), le butyl méthoxydibenzoylméthane (CAS 70356-09-1), etc.

Les absorbants UV seront utilisés seuls ou sous la forme de combinaison dont le pourcentage total dans la formule peut aller de 0,1 à 1% en poids sec par rapport au poids total de la composition.

Parmi les additifs utilisables selon l'invention, on citera également les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0,1 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05% à 2% en poids sec par rapport au poids total de la composition. Parmi ces additifs « traitants », on citera :
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Un aspect supplémentaire de l'invention concerne, l'utilisation d'une résine de copolymères d'acide adipique, d'acide fumarique, de néopentylglycol et de cyclohexanediméthanol, de préférence une résine de Polyester-26, dans une composition anhydre pour vernis à ongles, pour améliorer la propriété d'étalement et/ou d'application de ladite composition. En particulier, cet effet est particulièrement significatif lorsque la quantité de résine de copolymères d'acide adipique, d'acide fumarique, de néopentylglycol et de cyclohexanediméthanol, en particulier la résine de Polyester-26, est comprise de 0,1% à 5% en poids sec et de préférence de 0,3 % à 3 % en poids sec par rapport au poids total de la composition. De préférence, la résine de copolymères d'acide adipique, d'acide fumarique, de néopentylglycol et de cyclohexanediméthanol, en particulier la résine de Polyester-26 est solubilisée dans un solvant. Ce solvant est avantageusement l'acétate de butyle. Avantageusement, la résine est solubilisée dans le solvant, dans les proportions suivantes :
- 60% en poids de résine de copolymères d'acide adipique, d'acide fumarique, de néopentylglycol et de cyclohexanediméthanol, de préférence de résine de Polyester-26 et
- 40% en poids de solvant, de préférence de l'acétate de butyle, par rapport au poids total de la résine solubilisée dans le solvant.

Un autre objet de l'invention concerne un vernis à ongles comprenant une composition anhydre telle que décrite précédemment.

L'invention concerne également un procédé de préparation de la composition anhydre pour vernis à ongles. Ce procédé de préparation comprend l'étape de mélange d'au moins un solvant organique cosmétiquement acceptable, un agent filmogène et une résine de copolymères d'acide adipique, d'acide fumarique, de néopentylglycol et de cyclohexanediméthanol, en particulier une résine de Polyester-26, optionnellement avec d'autres additifs. De préférence, la résine de copolymères d'acide adipique, d'acide fumarique, de néopentylglycol et de cyclohexanediméthanol, en particulier la résine de Polyester-26 est solubilisée dans de l'acétate de butyle. Avantageusement, la résine est solubilisée dans le solvant, dans les proportions suivantes :
- 60% en poids de résine de copolymères d'acide adipique, d'acide fumarique, de néopentylglycol et de cyclohexanediméthanol, de préférence de résine de Polyester-26 et
- 40% en poids de solvant, de préférence de l'acétate de butyle, par rapport au poids total de la résine solubilisée dans le solvant.

Tous les modes de réalisation cités précédemment peuvent être combinés sous réserve de leur faisabilité technique.

### Exemples

Les exemples, ci-dessous, concernent diverses formulations de compositions anhydres pour vernis à ongles.

Cinq formulations distinctes ont été évaluées par un panel d'utilisateurs. En particulier, les propriétés d'étalement et d'application ont été appréciées par ce panel d'utilisateurs. Le panel d'utilisateurs a attribué une note à chacune de ces formulations :
- 1 étant la note la plus faible et correspondant à un résultat médiocre,
- 5 étant la note la plus élevée et correspondant à un résultat excellent.

Les utilisateurs ont appliqué chaque composition sur leurs ongles afin d'en estimer leurs propriétés.

La composition correspondant à la base dite standard ne comprend pas de résine de Polyester-26 solubilisée dans de l'acétate de butyle. Les compositions correspondant aux exemples 1-4 comprennent diverses concentrations de résine de Polyester-26 solubilisée dans de l'acétate de butyle.

**Tableau 1**

| **Ingrédients** | **fonction** | **Base standard** | **Ex 1** | **Ex 2** | **Ex 3** | **Ex 4** |
|---|---|---|---|---|---|---|
| Acétate d'éthyle | Solvant organique | 39,4 | 39,4 | 39,4 | 39,4 | 39,4 |
| Acétate de butyle | Solvant organique | 24,5 | 24,5 | 24,5 | 24,5 | 24,5 |
| Acétyl Tri-butyl Citrate | Plastifiant | 6 | 6 | 6 | 6 | 6 |
| Alcool iso-propylique (provenant de la nitrocellulose) | Solvant | 5,6 | 5,6 | 5,6 | 5,6 | 5,6 |
| Nitrocellulose (en poids sec) | Filmogène | 13 | 13 | 13 | 13 | 13 |
| Phthalic Anhydride / Trimellitic Anhydride / Glycols copolymer (en poids sec) | Résine | 9,5 | 8,9 | 8,3 | 7,7 | 7,1 |
| Bentonite | Agent thixotropant | 2 | 2 | 2 | 2 | 2 |
| Polyester-26 (en poids sec) | Agent d'application et d'étalement | 0 | 0,6 | 1,2 | 1,8 | 2,4 |
| **Total (% en poids)** | | 100 | 100 | 100 | 100 | 100 |
| Propriétés sensorielles évaluées par le Panel d'utilisateurs - Notation sensorielle : 1 = médiocre 5 = excellent | | | | | | |
| Facilité d'étalement | | 3 | 3,5 | 4 | 4,5 | 4,5 |
| Application | | 3 | 3,5 | 4 | 4,5 | 4,5 |

Il apparaît que les exemples 1-4 comprenant de la résine de Polyester-26 solubilisée dans de l'acétate de butyle obtiennent des notes supérieures en termes de facilité d'étalement et d'application en comparaison avec la base standard. De surcroît, on peut constater que ces notes augmentent avec la quantité de résine de Polyester-26 solubilisée dans de l'acétate de butyle ajoutée.

Il faut souligner également que l'ajout de la résine de Polyester-26 solubilisée dans de l'acétate de butyle n'a pas affecté les autres propriétés qui sont essentielles pour les utilisateurs à savoir, la brillance, la tenue, la couvrance ainsi que les temps de séchage et de durcissement.

Les résultats démontrent que l'ajout de résine de Polyester-26 solubilisée dans de l'acétate de butyle dans une composition anhydre pour vernis à ongles permet d'améliorer significativement ses propriétés d'étalement et d'application rendant sa pose facile et rapide.

## Revendications

1. Composition anhydre pour vernis à ongles comprenant au moins un solvant organique cosmétiquement acceptable, au moins un agent filmogène et une résine de copolymère(s) d'acide adipique, d'acide fumarique, de néopentylglycol et de cyclohexanediméthanol.

2. Composition anhydre pour vernis à ongles selon la revendication 1, **caractérisée en ce que** la résine de copolymère(s) d'acide adipique, d'acide fumarique, de néopentylglycol et de cyclohexanediméthanol est la résine de Polyester-26 (désignation INCI).

3. Composition anhydre pour vernis à ongles selon la revendication 1 ou 2, **caractérisée en ce que** la résine de copolymère(s) d'acide adipique, d'acide fumarique, de néopentylglycol et de cyclohexanediméthanol, de préférence la résine de Polyester-26, est solubilisée dans de l'acétate de butyle.

4. Composition anhydre pour vernis à ongles selon l'une quelconque des revendications précédentes, **caractérisée en ce que** la quantité de résine de copolymère(s) d'acide adipique, d'acide fumarique, de néopentylglycol et de cyclohexanediméthanol, est comprise entre 0,1 à 5% en poids sec par rapport au poids total de la composition anhydre.

5. Composition anhydre pour vernis à ongles selon l'une quelconque des revendications précédentes, **caractérisée en ce que** l'agent filmogène est choisi parmi la cellulose, un dérivé de cellulose, une résine vinylique, une résine acrylique ou n'importe quelle combinaison de ceux-ci.

6. Composition anhydre pour vernis à ongles selon l'une quelconque des revendications précédentes, **caractérisée en ce que** l'agent filmogène est la nitrocellulose.

7. Composition anhydre pour vernis à ongles selon l'une quelconque des revendications précédentes, **caractérisée en ce que** la quantité d'agent filmogène est comprise de 10 % à 20 % en poids sec par rapport au poids total de la composition anhydre pour vernis à ongles.

8. Composition anhydre pour vernis à ongles selon l'une quelconque des revendications précédentes, **caractérisée en ce que** le solvant organique est choisi parmi le toluène, le xylène, l'acétate d'éthyle, l'acétate de butyle, l'acétate d'isobutyle, l'acétate de propyle, l'acétate de méthyle une cétone, un ester, l'isopropanol, le butanol le diacétone alcool, l'éthanol dénaturé avec la méthylethylcétone ou le diethylether, les hydrocarbures linéaires ou cycliques, comme par exemple l'hexane, le cyclohexane, l'heptane ou n'importe quelle combinaison de ceux-ci.

9. Composition anhydre pour vernis à ongles selon l'une quelconque des revendications précédentes, **caractérisée en ce que** le solvant organique est l'acétate d'éthyle, l'acétate de butyle ou une combinaison de ceux-ci.

10. Composition anhydre pour vernis à ongles selon l'une quelconque des revendications précédentes, **caractérisée en ce que** la quantité de solvant organique est comprise de 60% à 80% par rapport au poids total de la composition anhydre pour vernis à ongles.

11. Composition anhydre pour vernis à ongles selon l'une quelconque des revendications précédentes comprenant en outre un ou plusieurs additifs choisis parmi les agents filmogènes, les résines, les plastifiants, les agents rhéologiques, les absorbeurs UV, les modificateurs de surface et les agents dits « traitants ».

12. Composition anhydre pour vernis à ongles selon l'une quelconque des revendications précédentes **caractérisée en ce qu'**elle comprend en outre un ou plusieurs agents de coloration choisis parmi les pigments, les nacres, les glitters et/ou les particules métalliques.

13. Vernis à ongles comprenant une composition anhydre selon l'une quelconque des revendications précédentes.

14. Utilisation d'une résine de copolymère(s) d'acide adipique, d'acide fumarique, de néopentylglycol et de cyclohexanediméthanol, de préférence une résine de Polyester-26, dans une composition anhydre pour vernis à ongles pour améliorer la propriété d'étalement et/ou d'application de ladite composition anhydre pour vernis à ongles.

15. Utilisation selon la revendication précédente **caractérisée en ce que** la quantité de résine de copolymère(s) d'acide adipique, d'acide fumarique, de néopentylglycol et de cyclohexanediméthanol, est comprise de 0,1 à 5% en poids sec par rapport au poids total de la composition anhydre pour vernis à ongles.

16. Procédé de préparation d'une composition anhydre pour vernis à ongles selon l'une des revendications 1 à 12 comprenant une étape de mélange d'au moins un solvant organique cosmétiquement acceptable, d'au moins un agent filmogène et d'une résine de copolymère(s) d'acide adipique, d'acide fumarique, de néopentylglycol et de cyclohexanediméthanol, de préférence d'une résine de Polyester-26.
